# EUROPÄISCHE PATENTSCHRIFT

(11) **EP 1 088 533 B1**
(45) Veröffentlichungstag und Bekanntmachung des Hinweises auf die Patenterteilung: **10.11.2004**
(21) Anmeldenummer: 00810773.2
(22) Anmeldetag: 29.08.2000
(51) Int. Cl.: A61F 2/44, A61F 2/28

(54) **Korbartiger Behälter zum implantieren von Knochengewebe**
Basket-style container for implanting bone tissue
Récipient du type corbeille pour implanter du tissue osseux

(30) Priorität: 28.09.1999 EP 99810873
(43) Veröffentlichungstag der Anmeldung: 04.04.2001
(73) Patentinhaber: Centerpulse Orthopedics Ltd., 6340 Baar (CH)
(72) Erfinder: Studer, Armin, 6312 Steinhausen (CH); Bollinger, Thomas, 8902 Urdorf (CH)
(74) Vertreter: Manitz, Finsterwald & Partner Gbr

(56) Entgegenhaltungen:
- EP-A- 0 268 115
- WO-A-94/18913
- WO-A-97/23175
- WO-A-98/26725
- WO-A-99/32055
- FR-A- 2 726 994
- US-A- 4 064 567

## Beschreibung

Die Erfindung betrifft einen korbartigen Behälter zum Implantieren von Knochengewebe gemäss Oberbegriff von Anspruch 1.

Es ist bekannt, chirurgische Eingriffe an Wirbelsäulen durchzuführen, um defekte Bandscheiben durch Implantate zu ersetzen. Mit solchen Implantaten, die Abstandhalteelemente zwischen Wirbeln bilden, werden benachbarte Wirbel steif verbunden. Es ist auch bekannt, einem Patienten Knochengewebe zu entnehmen, um dieses zu Rekonstruktionszwecken an einer anderen Stelle zu implantieren, wo das Gewebe dank seiner regenerativen Kräfte zu einem steifen Körper zusammenwächst. Bei Wirbelsäulen ist es vorteilhaft, wenn die genannten Abstandhalteelemente zumindest teilweise mit Knochengewebe hergestellt werden, wobei sich dessen regenerativen Kräfte nutzen lassen.

Die EP-A-0 268 115 offenbart einen zylinderförmigen Platzhalter als Wirbelersatz, der aus Streckmetall oder aus einem Blech mit.ausgestanzten Durchbrüchen entsteht, welches in Form eines Zylindermantels eingerollt ist. Wenn ein solches Element als Ersatz für einen Wirbel oder eine Bandscheibe eingesetzt wird, muss es eine bestimmte Mindeststeifigkeit aufweisen, die durch die Abmessungen der Stege und durch die Eigenschaften des Blechmaterials gegeben ist, aus dem die Durchbrüche herausgestanzt sind. Je schlechter die mechanischen Eigenschaften des Blechmaterials sind, desto kleiner müssen die Durchbrüche im Verhältnis zur Stegdicke sein.

Aufgabe der Erfindung ist es, ein Implantat zu schaffen, mit dem Knochengewebe eines Patienten in oder zwischen Knochen des Patienten implantierbar ist. Ausserdem muss ein Diagnoseverfahren realisierbar sein, mit dem ein Fortschritt einer Knochengewebebildung kontrollierbar ist. Diese Aufgabe wird durch den im Anspruch 1 definierten Behälter gelöst.

Der korbartige Behälter enthält ein Aufnahmevolumen für Knochengewebe. Nach einem Einfüllen des Knochengewebes wird der Behälter implantiert. Das Aufnahmevolumen befindet sich innerhalb einer um eine Achse angeordneten Wand. Diese periphere Wand besteht aus einem Gitter, Gewebe oder Geflecht. Quer zur Achse ermöglicht die Wand eine röntgenoptische Durchsicht des ungefüllten Aufnahmevolumens. Der Flächenanteil, der für Röntgenstrahlen durchlässig ist, beträgt mindestens 30%.

Die abhängigen Ansprüche 2 bis 8 betreffen vorteilhafte Ausführungsformen des erfindungsgemässen Behälters. Zwei Verwendungsmöglichkeiten des erfindungsgemässen Behälters sind nachstehend angegeben.

Nachfolgend wird die Erfindung anhand der Zeichnungen erläutert. Es zeigen:
- Fig. 1: eine perspektivische Darstellung eines erfindungsgemässen Behälters,
- Fig. 2: zwei Wirbel einer Wirbelsäule mit zwischen ihnen angeordneten Behältern gemäss der Erfindung,
- Fig. 3: eine zweite Ausführungsform des erfindungsgemässen Behälters,
- Fig. 4: schematische Querschnitts-Darstellung einer Behälterwand,
- Fig. 5a, 5b: entsprechende Querschnitte von zweikammerigen Behältern,
- Fig. 6: eine weitere Ausführungsform des erfindungsgemässen Behälters,
- Fig. 7: einen Querschnitt durch eine nahtlose Behälterwand,
- Fig. 8: ein Schrägbild zur Veranschaulichung des Aufbaus der nahtlosen Behälterwand und
- Fig. 9: eine Seitenansicht von einer Behälterwand mit dem Aufbau gemäss Fig. 8.

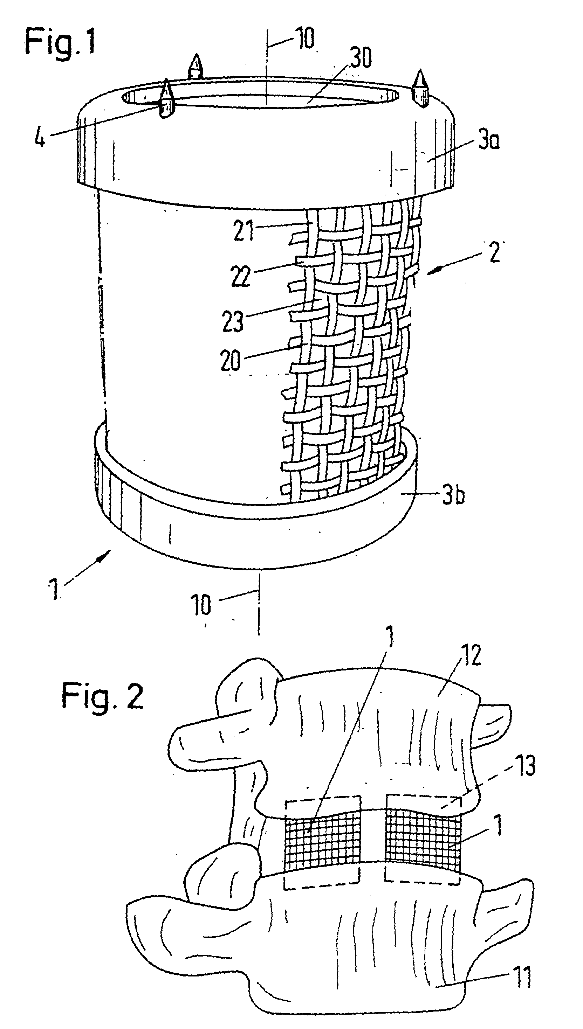

Ein erfindungsgemässer Behälter 1, wie er in Fig. 1 perspektivisch dargestellt ist, wird bei einem chirurgischen Eingriff, der an der Wirbelsäule eines Patienten vorgenommen wird, als versteifendes und abstandhaltendes Element zwischen Wirbel implantiert. Vor dem Implantieren wird der korbartige Behälter 1 mit Knochengewebe gefüllt, das dem Patienten an einer anderen Stelle entnommen worden ist. Das Aufnahmevolumen für das Knochengewebe befindet sich innerhalb einer um eine Achse 10 angeordneten Wand 2. Diese periphere Wand 2 - in Form eines Gitters, Gewebes oder Geflechts - ist beispielsweise aus Drähten 21, 22 aufgebaut, die in Bindungspunkten 20 verkreuzt sind. Die Drähte 21 einer ersten Schar sind achsparallel ausgerichtet; die restlichen Drähte 22, die eine zweite Schar bilden, sind zu jenen der ersten Schar quer gerichtet. Zwischen den Drähten 21, 22 sind relativ grosse Öffnungen 23 vorgesehen. Diese ermöglichen quer zur Achse 10 eine röntgenoptische Durchsicht des ungefüllten Aufnahmevolumens, die erfindungsgemäss mindestens 30% beträgt. Der Verbund aller Drähte 21, 22 soll eine ausreichende Stützfunktion ausüben, so dass - um eine dichte Packung erzeugen zu können - das Knochengewebe in das Aufnahmevolumen hineinpressbar ist.

Die röntgenoptische Durchsicht erlaubt es, bei Kontrolluntersuchungen nach einer Implantation den regenerativen Fortschritt des im Behälter 1 enthaltenen Gewebes zu überwachen.

Die Wand 2 ist an einem zur Achse 10 quer verlaufenden Rand von Ringen 3a und 3b begrenzt. Auf diesen Ringen sind Spitzen 4 angeordnet, die beim Implantieren eine Verankerung in den zu behandelnden Wirbeln ermöglichen. Der obere Ring 3a enthält eine Öffnung 30, durch die das Knochengewebe in das Aufnahmevolumen eingefüllt werden kann. Auch der untere Ring 3b kann eine entsprechende Öffnung aufweisen. Der Behälter 1 hat jedoch mit Vorteil am unteren Ring 3b einen Boden, der aus der gleichen Drahtstruktur wie die Wand 2 gebildet sein kann.

Fig. 2 zeigt einen ersten Lendenwirbel 11 einer Wirbelsäule, der mittels zweier Behälter 1 gemäss der Erfindung mit einem zweiten Lendenwirbel 12 verbunden ist. An den Wirbeln 11 und 12 kann, um Vertiefungen 13 für die Behälter 1 auszubilden, Knochengewebe entnommen sein.

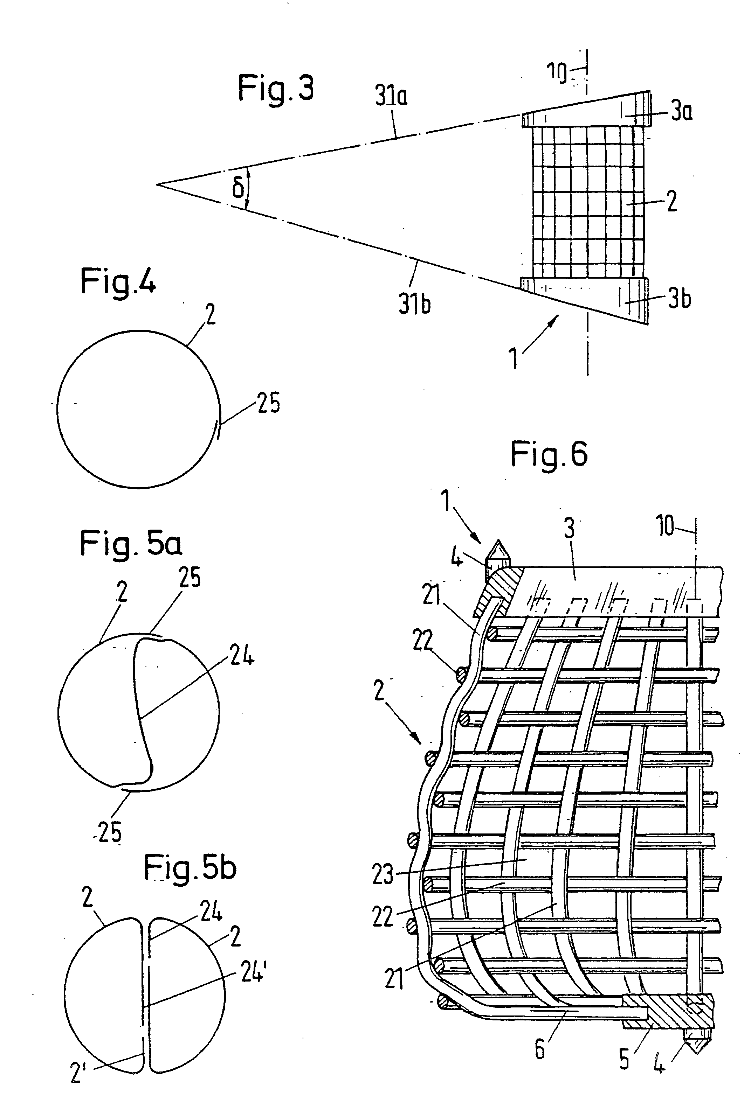

Die Ringe 3a und 3b, die bei der Ausführungsform gemäss Fig. 1 parallel zu einander angeordnet sind, können auch so ausgebildet sein, wie es in Fig. 3 dargestellt ist, nämlich so, dass die quer zur Achse 10 stehenden Aussenseiten der Ringe 3a und 3b auf Ebenen 31 a und 31 b liegen, die einen Winkel δ einschliessen. Bei chirurgischen Behandlungen von Lordose im Bereich der Lendenwirbelsäule können Behälter 1 gemäss der Fig. 3 eingesetzt werden.

Die Wand 2 kann - siehe Fig. 4 - einfach aus einem flachen Drahtgewebe hergestellt werden, indem dieses Gewebe zu einem Zylinder geformt und ein Überlappungsstreifen zu einer Naht 25 verschweisst wird. Bei der Ausführungsform der Fig. 5a ist ein flaches Drahtgewebe derart geformt, dass zwei Kammern sowie zwei Nähte 25 gebildet werden. Eine innere Wand 24 wirkt als Verstärkung der peripheren Wand 2. Eine andere zweikammerige Ausführungsform ist in Fig. 5b dargestellt. Hier sind zwei Rohrstücke 2' und 2" entlang einer ebenen Wand 24' zusammengefügt.

Die erfindungsgemässen Behälter 1 können auch als längere Rohre angeboten werden, deren Wände 2 so ausgebildet sind, dass sie auf eine erforderliche Länge zurecht geschnitten werden können. Dabei sind die Ringe 3a und 3b sowie die Wand 2 als separate Teile vorgesehen, die sich nach dem Zurechtschneiden der Wand 2 zusammenfügen lassen.

Bei den Ausführungsformen gemäss den Figuren 4, 5a und 5b sind die Wände 2 aus flachen Gewebestücken hergestellt. Es ist aber auch möglich, die Wand 2 als räumliches Flechtwerk auszubilden. Ein Beispiel ist in Fig. 6 als linke Hälfte eines Längsschnitts durch den Behälter 1 dargestellt. Vertikale Drähte 21 verbinden bogenförmig einen oben liegenden Eingangsring 3 mit einer am Boden 6 des Behälters 1 angeordneten Scheibe 5 und bilden dabei eine nach unten sich erweiternde Wand 2 sowie einen Teil des Bodens 6 aus. Quer zu den Drähten 21 sind Drähte 22 eingeflochten. Die Drähte 22 können geschlossene Ringe mit unterschiedlichen Durchmessern sein; sie können aber auch ein zusammenhängendes Stück bilden, das schraubenförmig zwischen die Drähte 21 eingeflochten ist. Am Eingangsring 3 und an der Bodenscheibe 5 sind Verankerungsspitzen 4 angebracht. Die Öffnungen 23 sind so gross ausgebildet, dass die erfindungsgemässe röntgenoptische Durchsicht quer zur Achse 10 möglich ist.

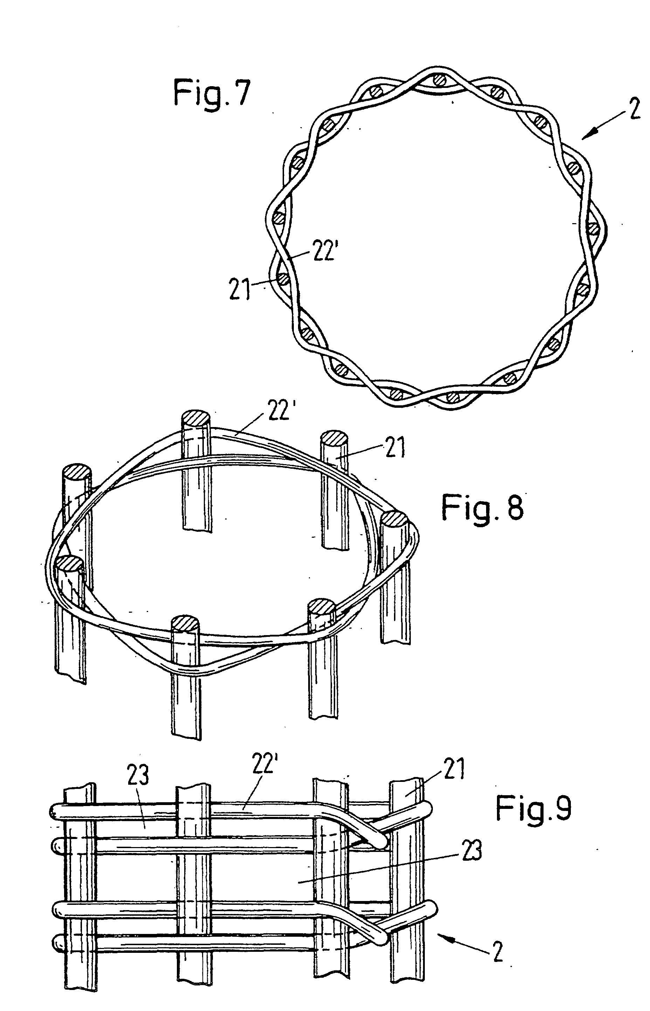

Der Behälter 1 der Fig. 6 hat eine nahtlose Wand 2. Ein weiteres Beispiel einer nahtlosen Wand 2 ist in Fig. 7 gezeigt. Die zwischen siebzehn (ungerade Anzahl) vertikale Drähte 21 eingeflochtenen Drähte 22' bilden Doppelringe, wie es in Fig. 8 für eine Ausführungsform mit nur sieben vertikalen Drähten 21 veranschaulicht ist. Damit eine solche nahtlose Ausführungsform möglich ist, muss die Anzahl der vertikalen Drähten 21 ungerade sein. Anhand der Seitenansicht in Fig. 9 mit zwei Doppelringen 22' ist illustriert, dass die Öffnungen 23 in der Wand 2 verschieden gross ausgebildet sein können.

Die Drähte 21 der ersten Schar weisen einen grösseren Durchmesser als die anderen Drähte 22 auf, damit sie - im Verbund mit den Drähten der zweiten Schar - einer grösseren Kraft in Richtung der Achse 10 standhalten können. Als Material können alle für Implantate geeigneten Metalle, insbesondere Titan, oder geeignete Legierungen verwendet werden. Ein Teil der Wände 2, der beispielsweise durch einzelne der Drähte 22 gebildet wird, kann auch nichtmetallisch sein und beispielsweise aus resorbierbarem Material bestehen.

Der Querschnitt des erfindungsgemässen Behälters 1 muss nicht notwendigerweise kreisförmig sein. Die Wand 2 kann beispielsweise die Form eines Vierkants oder Sechskants haben.

Ausser bei der Behandlung von Wirbelsäulen kann der erfindungsgemässe Behälter - falls geeignet geformt - für einen chirurgischen Eingriff an Knochen verwendet werden, um Gewebe zu substituieren, das beispielsweise aufgrund eines krebsartigen Tumors hat entfernt werden müssen.

Der erfindungsgemäße Behälter kann für chirurgische Eingriffe an Wirbelsäulen verwendet werden, wobei eine Bandscheibe durch mindestens einen mit Knochengewebe gefüllten Behälter, vorzugsweise zwei, substituiert wird, und wobei die Behälter eine versteifende und abstandhaltende Verbindung zwischen Wirbeln der Wirbelsäule herstellen.

Der erfindungsgemäße Behälter kann auch für chirurgische Eingriffe an Knochen verwendet werden, wobei mit dem gefüllten Behälter entferntes Gewebe substituiert wird.

## Patentansprüche

1. Korbartiger Behälter (1) zum Aufnehmen von Knochengewebe und anschliessenden Implantieren, mit einem Aufnahmevolumen für Knochengewebe, das sich innerhalb einer um eine Achse (10) angeordneten peripheren Wand (2) befindet, **dadurch gekennzeichnet,**
**dass** die Wand (2) aus einem Gitter, Gewebe oder Geflecht aus zumindest teilweise metallischen, in Bindungspunkten (20) verkreuzten Drähten (21, 22) besteht, das quer zur Achse einen Flächenanteil von mindestens 30% aufweist, der nicht durch die Drähte (21, 22) belegt ist, um den Fortschritt einer Knochengewebebildung im Aufnahmevolumen durch eine röntgenoptische Durchsicht kontrollierbar zu machen.

2. Behälter nach Anspruch 1, **dadurch gekennzeichnet, dass** Drähte (21) einer ersten Schar weitgehend achsparallel ausgerichtet sind, die restlichen Drähte (22), die eine zweite Schar bilden, zu jenen der ersten Schar quergerichtet sind, und dass der Verbund aller Drähte eine ausreichende Stützfunktion ausübt, so dass - zur Erzeugung einer dichten Packung - das Knochengewebe in das Aufnahmevolumen hineinpressbar ist.

3. Behälter nach Anspruch 1 oder 2, **dadurch gekennzeichnet, dass** die Drähte (21) der ersten Schar einen grösseren Durchmesser als die anderen Drähte (22) aufweisen, und dass sie im Verbund mit den Drähten der zweiten Schar einer vorgegebenen Kraft in Richtung der Achse (10) standhalten können.

4. Behälter nach einem der Ansprüche 1 bis 3, **dadurch gekennzeichnet, dass** die Wand (2) nahtlos ist.

5. Behälter nach einem der Ansprüche 1 bis 4, **dadurch gekennzeichnet, dass** eine Seite des Aufnahmevolumens quer zur Achse (10) einen Boden (6) aufweist.

6. Behälter nach einem der Ansprüche 1 bis 5, **dadurch gekennzeichnet, dass** eine Verstärkungswand (24, 24') innerhalb des Aufnahmevolumens die periphere Wand (2) verstärkend verbindet.

7. Behälter nach einem der Ansprüche 1 bis 6, **dadurch gekennzeichnet, dass** die Wände (2, 24) zumindest zum Teil aus Titan bestehen, und dass insbesondere in der Wand zu einem weiteren Teil resorbierbares Material enthalten ist.

8. Behälter nach einem der Ansprüche 1 bis 7, **dadurch gekennzeichnet, dass** die Wand (2) zumindest an einem zur Achse (10) quer verlaufenden Rand von einem Ring (3; 3a, 3b) begrenzt ist, und dass vorzugsweise auf diesem Ring Verankerungsspitzen (4) angeordnet sind.

## Claims

1. Basket-like container (1) for the reception of bone tissue and for subsequent implantation, comprising a reception volume for bone tissue which is located within a peripheral wall (2) arranged about an axis (10), **characterized in that** the wall (2) consists of a grid, of a fabric or of a mesh which is made of wires (21, 22) at least partly of metal and crossed at mesh nodes (20) and which has a component by area transverse to the axis of at least 30% not covered by the wires (21, 22) in order to make it possible to monitor the progress of bone tissue formation in the reception volume by an X-ray optical inspection.

2. Container in accordance with claim 1, **characterized in that** wires (21) of a first array are oriented largely parallel to the axis and the remaining wires (22), which form a second array, are directed transversely to those of the first array; and **in that** the assembly of all wires provides a sufficient support function that the bone tissue can be pressed into the reception volume to produce a dense packing.

3. Container in accordance with claim 1 or claim 2, **characterized in that** the wires (21) of the first array have a larger diameter than the other wires (22); and **in that** they can withstand a predetermined force in the direction of the axis (10) in an assembly with the wires of the second array.

4. Container in accordance with any one of the claims 1 to 3, **characterized in that** the wall (2) is seamless.

5. Container in accordance with any one of the claims 1 to 4, **characterized in that** a side of the reception volume transverse to the axis (10)has a base (6).

6. Container in accordance with any one of the claims 1 to 5, **characterized in that** a reinforcing wall (24, 24') within the reception volume connects the peripheral wall (2) in a reinforcing manner.

7. Container in accordance with any one of the claims 1 to 6, **characterized in that** the walls (2, 24) consist at least partly of titanium; and **in that** a further part of the material contained in the wall is in particular an absorbable material.

8. Container in accordance with any one of the claims 1 to 7, **characterized in that** the wall (2) is bounded by a ring (3; 3a, 3b) at least at a rim which extends transversely to the axis (10); and **in that** anchoring points (4) are preferably arranged on this ring.

## Revendications

1. Récipient (1) du type corbeille pour recevoir du tissu osseux et pour une implantation successive, présentant un volume de réception de tissu osseux situé à l'intérieur d'une paroi périphérique (2) agencée autour d'un axe (10), **caractérisé en ce que**
la paroi (2) consiste en un grillage, en un tissage ou en un tressage qui est constitué par des fils (21, 22) au moins partiellement métalliques, entrecroisés en des points de liaison (20), et qui présente, transversalement par rapport à l'axe, une part de superficie d'au moins 30 % non occupée par les fils (21, 22), de façon à pouvoir contrôler la progression d'une formation de tissu osseux, dans le volume de réception, par observation radiographique.

2. Récipient selon la revendication 1, **caractérisé en ce que** des fils (21) d'une première nappe sont majoritairement orientés parallèlement à l'axe, les fils (22) restants, formant une seconde nappe, étant orientés transversalement par rapport à ceux de la première nappe ; et **en ce que** l'assemblage de tous les fils exerce une fonction de soutien suffisante, de telle sorte que le tissu osseux puisse être enfoncé dans le volume de réception - en vue d'engendrer un regroupement compact.

3. Récipient selon la revendication 1 ou 2, **caractérisé en ce que** les fils (21) de la première nappe présentent un plus grand diamètre que les autres fils (22) ; et **en ce que** qu'ils peuvent résister à une force préétablie, dans la direction de l'axe (10), en association avec les fils de la seconde nappe.

4. Récipient selon l'une des revendications 1 à 3, **caractérisé en ce que** la paroi (2) est dépourvue de couture.

5. Récipient selon l'une des revendications 1 à 4, **caractérisé en ce que** qu'un côté du volume de réception comporte un fond (6) transversal par rapport à l'axe (10).

6. Récipient selon l'une des revendications 1 à 5, **caractérisé en ce que** qu'une paroi de renfort (24, 24') solidarise la paroi périphérique (2), à l'intérieur du volume de réception, avec effet de renforcement.

7. Récipient selon l'une des revendications 1 à 6, **caractérisé en ce que** les parois (2, 24) consistent au moins partiellement en du titane ; et **en ce que** notamment la paroi renferme un matériau résorbable, pour une part restante.

8. Récipient selon l'une des revendications 1 à 7, **caractérisé en ce que** la paroi (2) est délimitée par une bague (3 ; 3a, 3b), au moins sur un bord s'étendant transversalement par rapport à l'axe (10) ; et **en ce que** des picots d'ancrage (4) sont de préférence disposés sur cette bague.
